# EUROPEAN PATENT SPECIFICATION

(11) **EP 1 954 177 B1**
(45) Date of publication and mention of the grant of the patent: **18.03.2009**
(21) Application number: 06821419.6
(22) Date of filing: 13.11.2006
(51) Int. Cl.: G01N 21/47, G01N 21/64

(54) **A DEVICE FOR IMAGING AN INTERIOR OF A TURBID MEDIUM**
VORRICHTUNG ZUR ABBILDUNG DES INNEREN EINES TRÜBEN MEDIUMS
DISPOSITIF D'IMAGERIE DE L'INTERIEUR D'UN MILIEU TROUBLE

(30) Priority: 23.11.2005 EP 05111164
(43) Date of publication of application: 13.08.2008
(73) Proprietor: Koninklijke Philips Electronics N.V., 5621 BA Eindhoven (NL)
(72) Inventor: BAKKER, Levinus, P., NL-5656 AA Eindhoven (NL); VAN BEEK, Michael, C., NL-5656 AA Eindhoven (NL); VAN DER MARK, Martinus, B., NL-5656 AA Eindhoven (NL)
(74) Representative: Schouten, Marcus Maria
(86) International application number: PCT/IB2006/054226
(87) International publication number: WO 2007/060571

(56) References cited:
- WO-A-95/02987
- US-A1- 2002 045 833

## Description

The invention relates to a device for imaging an interior of a turbid medium, said device comprising:
a) a measurement volume for accommodating the turbid medium;
b) a light source for irradiating the turbid medium;
c) coupling means for coupling light from the light source into the measurement volume;
d) detection means for detecting light emanating from the measurement volume as a result of the irradiation of the turbid medium;
e) image reconstruction means for determining an image of the interior of the turbid medium from the detected light;
f) the light source is arranged for irradiating the turbid medium with light having a wavelength that is chosen such that the light causes fluorescent emission in a contrast agent in the turbid medium;
g) the detection means is arranged for detecting the fluorescent emission in at least two spectral regions; and
h) the image reconstruction means is arranged for performing image reconstruction calculations for the at least two spectral regions detected.

A device with these features is known from US patent application US 2002/045833 A. This device is used in a medical imaging system adapted to acquire images of the interior of a turbid medium, such as a human breast.

A device having features a) to e) is known from US patent 6,327,488 B1. The known device can be used for imaging an interior of a turbid medium, such as biological tissues. In medical diagnostics the device may be used for imaging an interior of a female breast. A turbid medium, such as a breast, is accommodated inside the measurement volume and irradiated with light from the light source, such as a laser. The measurement volume may be bounded by a holder having only one open side, with the open side being bounded by an edge portion. This edge portion may be provided with an elastically deformable sealing ring. Such a holder is known from US patent 6,480,281 B1. Light emanating from the measurement volume is detected and is used to derive an image of the interior of the turbid medium.

It is a drawback of the known device that it involves the use of multiple light sources, each producing light with a different wavelength and each requiring its own series of measurements, when the known device is used in medical diagnostics, for example for imaging biological tissues, performing spectrographic measurements, for example for obtaining information on the basis of which the nature of lesions can be determined.

It is an object of the invention to obtain more easily spectrographic information in a measurement that may be used in determining the nature of detected lesions.

According to the invention, this object is realized in that the device is characterized in that:
i) a means is provided for determining a local concentration of a predetermined component present in the turbid medium from the local absorption of fluorescent light in the at least two spectral regions of the fluorescent emission.

The invention also relates to medical image acquisition device comprising the device.

The invention is based on the recognition that a component present in the turbid medium may selectively absorb light from different parts of the spectrum of the fluorescent light present in the turbid medium. This means that the concentration of a predetermined component present in the turbid medium can be determined through detection of the local absorption of the fluorescent light in at least two spectral regions. With the proper predetermined component, the concentration of the predetermined component gives additional information that may be used in determining the nature of detected lesions.

Spectroscopic analysis of absorption spectra is mentioned in US patent 6,694,159 B2. However, this analysis does not concern light emitted by a fluorescent agent, but multi-wavelength light from an external light source not present in the turbid medium.

An embodiment of the device according to the invention is characterized in that
the predetermined component is chosen from the group comprising oxyhaemoglobin and deoxyhaemoglobin. This embodiment has the advantage that in medical diagnostics, where the device may be used for imaging an interior of a female breast, a determination of the concentrations of substances like oxyhaemoglobin and deoxyhaemoglobin can provide information on the oxygenation of lesions. Tumors, including breast tumors, have the characteristic that cell division takes place at a rapid pace requiring a lot of energy. Hence tumors are supplied with blood by a network of blood vessels having a density that is about double that of a network of blood vessels in healthy tissue. Moreover, as cell division takes place rapidly, blood in and around a tumor will contain less oxygen than blood in healthy tissue because the oxygen is used in the cell division process. Therefore, information on the oxygenation of lesions, which may be potential tumors, can be helpful in determining the nature of such lesions.

The invention also relates to a medical image acquisition device comprising the device according to any one of the embodiments described above.

These and other aspects of the invention will be further elucidated and described with reference to the drawings, in which:
Fig. 1 schematically shows an embodiment of the device for imaging an interior of a turbid medium.
Fig. 2, comprising Figs. 2a, 2b, 2c, 2d, and 2e, illustrates how the concentrations of oxyhaemoglobin and deoxyhaemoglobin can be determined from a measurement of the transmission of light emitted by a fluorescent contrast agent in a turbid medium.
Fig. 2a schematically shows the emission spectrum of a fluorescent contrast agent.
Fig. 2b schematically shows the absorption spectrum of oxyhaemoglobin.
Fig. 2c schematically shows the absorption spectrum of deoxyhaemoglobin.
Fig. 2d schematically shows a combined absorption spectrum of oxyhaemoglobin and deoxyhaemoglobin.
Fig. 2e schematically shows the transmission spectrum resulting from combining the emission spectrum of a fluorescent contrast agent as shown in Fig. 2a with the combined absorption spectrum of oxyhaemoglobin and deoxyhaemoglobin as shown in Fig. 2d.
Fig. 3 shows an embodiment of a medical image acquisition device according to the invention.

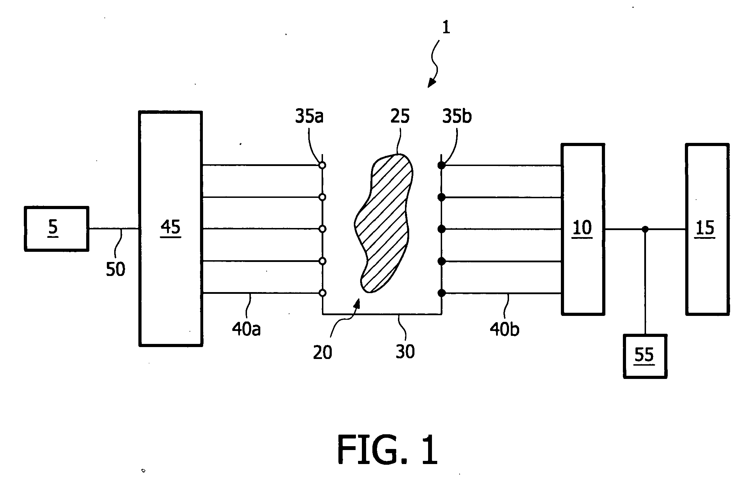

Fig. 1 schematically shows an embodiment of a device according to the invention for imaging an interior of a turbid medium as known from the prior art. The device I includes a light source 5, a photodetector unit 10, an image reconstruction unit 15, a measurement volume 20 for receiving a turbid medium 25, said measurement volume 20 being bounded by a receptacle 30, said receptacle comprising a plurality of entrance positions for light 35a and a plurality of exit positions for light 35b, and light guides 40a and 40b coupled to said entrance and exit positions for light. The device 1 further includes a selection unit 45 for coupling an input light guide 50 to a number of selected entrance positions for light 35a in the receptacle. For the sake of clarity, entrance positions for light 35a and exit positions for light 35b have been positioned at opposite sides of the receptacle 30. In reality, however, both types of positions may be distributed around the measurement volume 20. A turbid medium 25 is accommodated inside the measurement volume 20. The turbid medium 25 is irradiated with light from the light source 5 from a plurality of positions in that the light source 5 is coupled by means of the selection unit 45 to successively selected entrance positions for light, said selected entrance positions for light being selected from the plurality of entrance positions for light 35a. Light emanating from the measurement volume 20 is detected from a plurality of positions through further exit positions for light from among the plurality of exit positions for light 35b by means of photodetector unit 10. The image reconstruction unit 15 then uses the detected light to derive an image of an interior of the turbid medium 25. According to the invention, the light source 5 is arranged such that the turbid medium 25 can be irradiated with light having a wavelength that is chosen so as to cause fluorescent emission in a contrast agent. Further according to the invention, the photodetector unit 10 in its turn is further arranged to detect the fluorescent emission in at least two spectral regions. According to the invention, the device 1 still further comprises means 55 for determining the local concentration of a predetermined component present in the turbid medium 25. These means may be incorporated into a computer unit. Still according to the invention, the image reconstruction means 15 may be arranged to perform image reconstruction calculations for the at least two spectral regions in which the fluorescent emission is detected. In medical diagnostics, where the device may be used to image female breasts to check for the presence of tumors, the additional information may also be used to help locate potential tumors. Information relating to the concentration of a predetermined component present in a potential tumor may be used to help determine the possible nature of such a potential tumor.

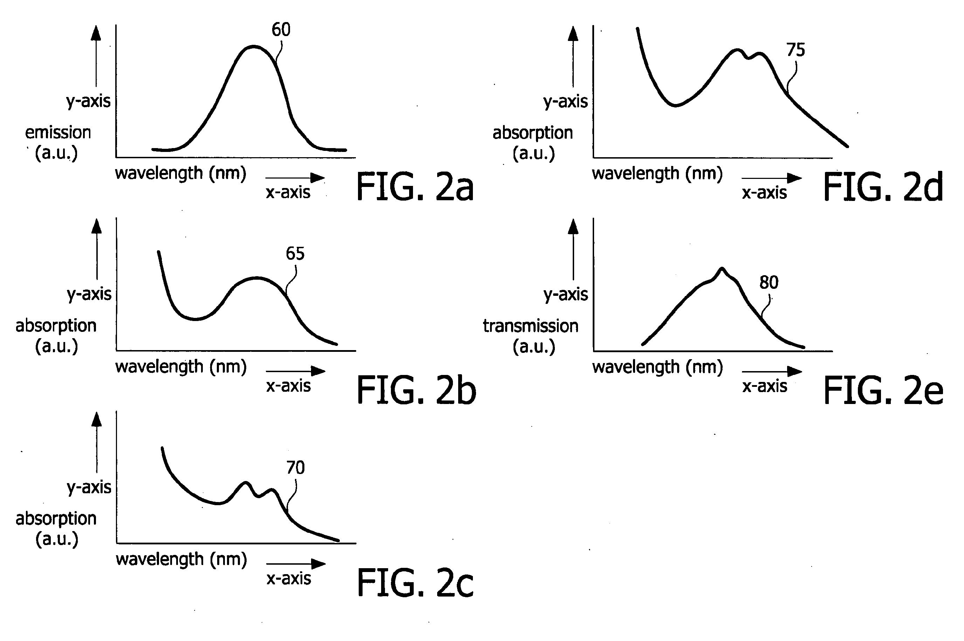

Fig. 2, comprising Figs. 2a, 2b, 2c, 2d, and 2e, illustrates how the concentrations of oxyhaemoglobin and deoxyhaemoglobin can be determined from a measurement of the transmission of light emitted by a fluorescent contrast agent in a turbid medium.

Fig. 2a schematically shows the emission spectrum, represented by curve 60, of a fluorescent contrast agent. Wavelength is plotted on the x-axis in nanometers, and emission is plotted on the y-axis in arbitrary units.

Fig. 2b schematically shows the absorption spectrum of oxyhaemoglobin represented by curve 65. Wavelength is plotted on the x-axis in nanometers, and absorption is plotted on the y-axis in arbitrary units. The values of curve 65 along the y-axis scale with the concentration of oxyhaemoglobin.

Fig. 2c schematically shows the absorption spectrum of deoxyhaemoglobin represented by curve 70. Wavelength is plotted on the x-axis in nanometers, and absorption is plotted on the y-axis in arbitrary units. The values of curve 70 along the y-axis scale with the concentration of deoxyhaemoglobin.

Fig. 2d schematically shows a combined absorption spectrum of oxyhaemoglobin and deoxyhaemoglobin represented by curve 75. Wavelength is plotted on the x-axis in nanometers, absorption is plotted on the y-axis in arbitrary units. As in Figs. 2b and 2c, the values of curve 75 along the y-axis scale with the concentration of oxyhaemoglobin and deoxyhaemoglobin. The shape of curve 75 is determined by the relative contributions of the absorption spectrums of oxyhaemoglobin in deoxyhaemoglobin shown in Fig. 2b and in Fig. 2c in curves 65 and 70, respectively. These relative contributions again depend on the concentrations of oxyhaemoglobin and deoxyhaemoglobin in the turbid medium imaged.

Fig. 2e schematically shows the transmission spectrum, represented by curve 80, resulting from combining the emission spectrum of a fluorescent contrast agent as shown in Fig. 2a by curve 60 with the combined absorption spectrums of oxyhaemoglobin and deoxyhaemoglobin as shown in Fig. 2d by curve 75. Wavelength is plotted on the x-axis in nanometers, transmission is plotted on the y-axis in arbitrary units. According to the invention, curve 80 is measured in at least two spectral regions. After curve 80 has been measured in at least two spectral regions, the values obtained are corrected for the emission spectrum of the fluorescent contrast agent as represented by curve 60 in Fig. 2a in that the obtained values are divided by the values of the emission spectrum of curve 60 in the same spectral regions. If the values obtained from curve 80 are values that result from integration of curve 80 over each of the at least two spectral regions, each value is divided by the value that results from a similar integration of curve 60 over the corresponding spectral region.

This essentially results in curve 75 shown in Fig. 2d sampled in at least two spectral regions. However, the important difference is that the information obtained from curve 80 in Fig. 2e is scaled with the concentrations of oxyhaemoglobin and deoxyhaemoglobin present in the turbid medium. Measuring curve 80 shown in Fig. 2e in at least two spectral regions, correcting the obtained data for the emission spectrum of the fluorescent contrast agent shown in Fig. 2a, and fitting the results to curves 65 and 70 shown in Figs. 2b and 2c, respectively, results in at least two equations with two variables. From these equations at least the relative concentrations of oxyhaemoglobin and deoxyhaemoglobin inside the turbid medium can be obtained. If the transmission spectrum is a result of absorption of fluorescent light by N components present in a turbid medium, performing the procedure described above for N spectral regions results in N equations with N variables. Then the N concentrations can be calculated from these N equations.

Up to this point in the explanation relating to Fig. 2, the obtained concentrations are averaged over the path traveled through the turbid medium by fluorescent light before being detected. However, as the turbid medium is imaged from a plurality of positions and as the image reconstruction process is able to provide an image of an interior of the turbid medium on the basis of light that has traveled through the turbid medium and is detected outside the turbid medium, the obtained information relating to concentrations can be calculated back into local concentrations that have not been averaged over the path length traveled.

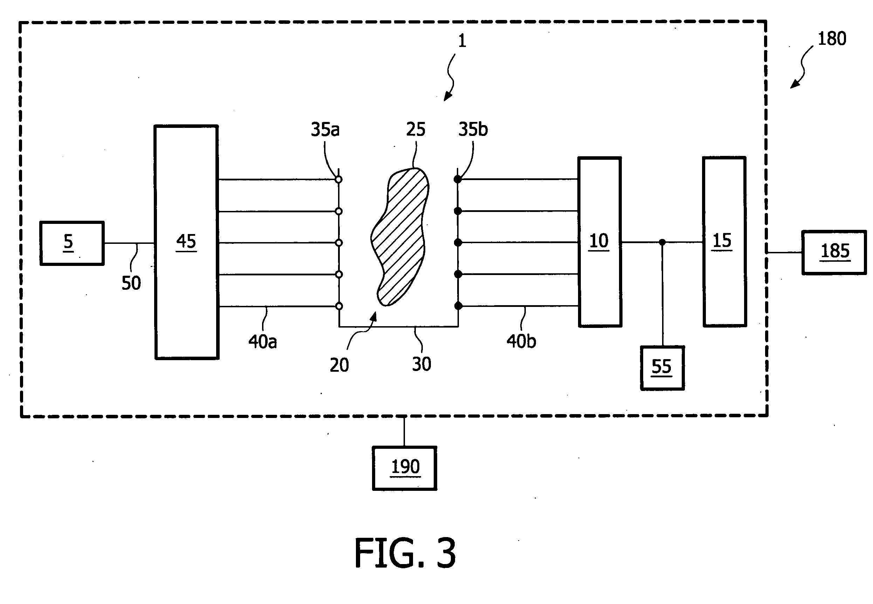

Fig. 3 shows embodiment of a medical image acquisition device according to the invention. The medical image acquisition device 180 comprises the device 1 discussed in fig. 1 as indicated by the dashed square. In addition to the device 1 the medical image acquisition device 180 further comprises a screen 185 for displaying an image of an interior of the turbid medium 25 and an input interface 190, for instance, a keyboard enabling and operated to interact with the medical image acquisition device 180.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. In the system claims enumerating several means, several of these means can be embodied by one and the same item of computer readable software or hardware. The mere fact that certain measures are recited in mutually different dependent claims does not indicate that a combination of these measures cannot be used to advantage.

## Claims

1. A device (1) for imaging an interior of a turbid medium (25) comprising:
a) a measurement volume (20) for accommodating the turbid medium (25);
b) a light source (5) for irradiating the turbid medium (25);
c) coupling means (40a, 50) for coupling light from the light source (5) into the measurement volume (20);
d) detection means (10) for detecting light emanating from the measurement volume (20) as a result of the irradiation of the turbid medium (25);
e) image reconstruction means (15) for determining an image of the interior of the turbid medium (25) from the detected light,
wherein
f) the light source (5) is arranged for irradiating the turbid medium (25) with light having a wavelength that is chosen such that the light causes fluorescent emission in a contrast agent in the turbid medium;
g) the detection means (10) is arranged for detecting the fluorescent emission in at least two spectral regions;
h) the image reconstruction means (15) is arranged for performing image reconstruction calculations for the at least two spectral regions detected;
**characterized in that**
i) a means (55) is provided for determining a concentration of a predetermined component present in the turbid medium (25) from the absorption of fluorescent light in the at least two spectral regions of the detected fluorescent emission.

2. A device (1) as claimed in claim 1, wherein the predetermined component is chosen from the group comprising oxyhaemoglobin and deoxyhaemoglobin.

3. A medical image acquisition device comprising the device according to any of the claims 1 and 2.

## Patentansprüche

1. Vorrichtung (1) zum Abbilden eines Inneren eines trüben Mediums (25), die Folgendes umfasst:
a) ein Messvolumen (20) zum Aufnehmen des trüben Mediums (25);
b) eine Lichtquelle (5) zum Bestrahlen des trüben Mediums (25);
c) Kopplungsmittel (40a, 50) zum Einkoppeln von Licht von der Lichtquelle (5) in das Messvolumen (20);
d) Detektionsmittel (10) zum Detektieren des aus dem Messvolumen (20) infolge der Bestrahlung des trüben Mediums (25) austretenden Lichts;
e) Bildrekonstruktionsmittel (15) zum Bestimmen eines Bildes des Inneren des trüben Mediums (25) ausgehend von dem detektierten Licht,
wobei
f) die Lichtquelle (5) vorgesehen ist, um das trübe Medium (25) mit Licht von einer Wellenlänge zu bestrahlen, die so gewählt wird, dass das Licht eine Fluoreszenzemission in einem Kontrastmittel in dem trüben Medium verursacht;
g) das Detektionsmittel (10) vorgesehen ist, um die Fluoreszenzemission in mindestens zwei Spektralbereichen zu detektieren;
h) das Bildrekonstruktionsmittel (15) vorgesehen ist, um Bildrekonstruktionsberechnungen für die mindestens zwei detektierten Spektralbereiche durchzuführen;
**dadurch gekennzeichnet, dass**
i) ein Mittel (55) vorgesehen ist, um eine Konzentration einer vorgegebenen, in dem trüben Medium (25) vorhandenen Komponente ausgehend von der Absorption des fluoreszenten Lichts in den mindestens zwei Spektralbereichen der detektierten Fluoreszenzemission zu ermitteln.

2. Vorrichtung (1) nach Anspruch 1, wobei die vorgegebene Komponente aus der Gruppe gewählt wird, die Oxyhämoglobin und Desoxyhämoglobin umfasst.

3. Medizinische Bilderfassungsvorrichtung, die die Vorrichtung nach einem der Ansprüche 1 und 2 umfasst.

## Revendications

1. Dispositif (1) d'imagerie d'un intérieur d'un milieu trouble (25) comprenant :
a) un volume de mesure (20) pour prendre en charge le milieu trouble (25) ;
b) une source de lumière (5) pour irradier le milieu trouble (25) ;
c) des moyens de couplage (40a, 50) pour coupler la lumière de la source de lumière (5) dans le volume de mesure (20) ;
d) des moyens de détection (10) pour détecter la lumière émanant du volume de mesure (20) sous l'effet de l'irradiation du milieu trouble (25) ;
e) des moyens de reconstruction d'image (15) pour déterminer une image de l'intérieur du milieu trouble (25) à partir de la lumière détectée ;
dans lequel
f) la source de lumière (5) est agencée pour irradier le milieu trouble (25) avec une lumière ayant une longueur d'onde qui est choisie de sorte que la lumière provoque une émission fluorescente dans un agent de contraste dans le milieu trouble ;
g) les moyens de détection (10) sont agencés pour détecter l'émission fluorescente dans au moins deux régions spectrales ;
h) les moyens de reconstruction d'image (15) sont agencés pour effectuer des calculs de reconstruction d'image pour les au moins deux régions spectrales détectées ;
**caractérisé en ce que**
i) un moyen (55) est fourni pour déterminer une concentration d'un composant prédéterminé présent dans le milieu trouble (25) à partir de l'absorption de lumière fluorescente dans les au moins deux régions spectrales de l'émission fluorescente détectée.

2. Dispositif (1) selon la revendication 1, dans lequel le composant prédéterminé est choisi dans le groupe se composant d'oxyhémoglobine et de déoxyhémoglobine.

3. Dispositif d'acquisition d'image médicale comprenant le dispositif selon l'une quelconque des revendications 1 et 2.
